(19) Europäisches Patentamt
European Patent Office
Office européen des brevets

(11) Publication number: 0 261 710 B1

# (12) EUROPEAN PATENT SPECIFICATION

(45) Date of publication of the patent specification: 23.05.90

(51) Int. Cl.[5]: C07D 307/20, C07D 309/10, A01N 47/30

(21) Application number: 87201613.4

(22) Date of filing: 26.08.87

## (54) Phenylurea herbicides.

(30) Priority: 28.08.86 US 901228

(43) Date of publication of application: 30.03.88 Bulletin 88/13

(45) Publication of the grant of the patent: 23.05.90 Bulletin 90/21

(84) Designated Contracting States: AT BE CH DE ES FR GB GR IT LI LU NL SE

(56) References cited:
EP-A- 0 136 702
DE-A- 2 917 914
FR-A- 2 471 979
GB-A- 2 115 418
US-A- 3 778 473
US-A- 3 914 300

(73) Proprietor: SHELL INTERNATIONALE RESEARCH MAATSCHAPPIJ B.V., Carel van Bylandtlaan 30, NL-2596 HR Den Haag(NL)

(72) Inventor: Pilgram, Kurt Hans Gerhard, 2607 Warwick Lane, Modesto California 95350(US)
Inventor: Bozarth, Gene Allen, 920 Eastridge Drive, Modesto California 95355(US)

(74) Representative: Bennett, David Arthur Horder et al, 4, York Road, London SE1 7NA(GB)

EP 0 261 710 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel oximinoalkyl phenylureas, their preparation, their use as herbicides and to herbicidal compositions containing these ureas.

It is known from U.S. Patent Nos. 3,778,473 and 3,914,300 that certain phenylurea oximes have fungicidal, herbicidal or plant growth regulator activity. However, it is desirable to have herbicides with a higher level of activity and a better spectrum of activity, particularly against troublesome broadleaf weed species including pigweed, velvetleaf, prickly sida, cotchweed bedstraw and spudwell. The present invention provides novel phenylurea oximes that are more efficient herbicides in activity and week sprectrum while showing greater selectivity to braodleaf crops such as cotton, soybeans and the like, particularly in pre-emergence application.

The present invention is directed to novel compounds of the Formula I

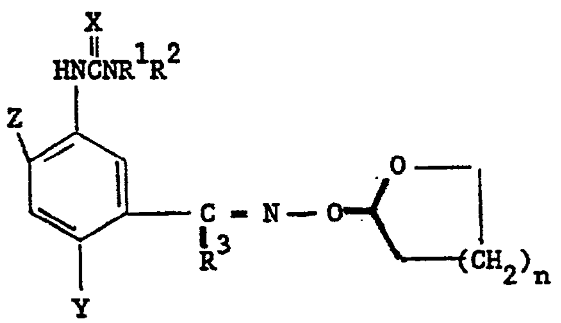

(I)

wherein $R^1$ and $R^2$ each independently is a hydrogen atom, cyclopropyl, or alkyl or alkoxy containing 1 to 5 carbon atoms; $R^3$ is hydrogen or alkyl or alkoxy containing 1 to 3 carbon atoms; X is O or S; Y is hydrogen, alkyl containing 1 to 4 carbon atoms, fluorine, chlorine or bromine; Z is hydrogen or fluorine; and n is 1 or 2. The compounds are useful as herbicides.

The oxime substituent group of the compounds of the invention gives rise to geometric isomerism by virtue of the presence of an asymmetrically substituted double bond. These isomers are usually described as follows:

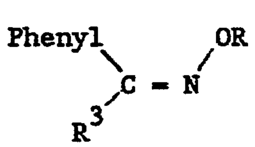

AND

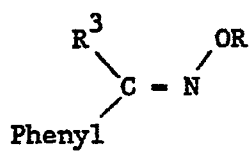

Syn- or Z- isomer

Anti- or E- isomer

The Formula I includes all the possible configurations as well as individual configurations or mixtures. The present invention includes all the herbicidally active forms resulting from synthesis, and of deliberately created mixtures of such forms.

Illustrative and representative species of the present invention include:

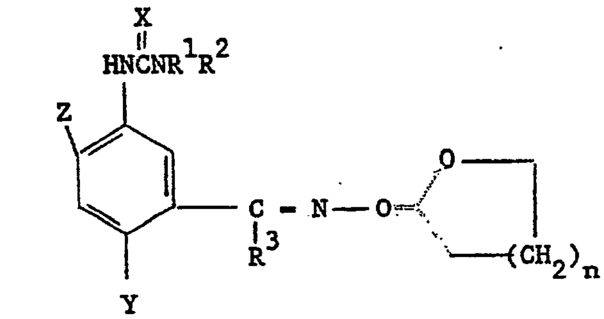

(I)

| X | $R^1$ | Y | Z | $R^2$ | $R^3$ | n |
|---|---|---|---|---|---|---|
| O | $CH_3$ | H | H | $CH_3$ | $OCH_3$ | 2 or 1 |
| O | $CH_3$ | H | F | $CH_3$ | $CH_3$ | 2 or 1 |
| O | H | $CH_3$ | H | $CH_3$ | $OCH_3$ | 2 or 1 |
| O | H | $CH_3$ | F | $CH_3$ | $CH_3$ | 2 or 1 |
| O | H | Cl | H | $CH_3$ | $OCH_3$ | 2 or 1 |
| O | H | Cl | F | $CH_3$ | $OCH_3$ | 2 or 1 |
| O | H | F | H | $CH_3$ | $OCH_3$ | 2 or 1 |
| O | $CH_3$ | F | H | $CH_3$ | $OCH_3$ | 2 or 1 |
| O | $CH_3$ | F | H | $CH_3$ | $CH_3$ | 2 or 1 |
| O | $CH_3$ | H | H | $CH_3$ | H | 2 or 1 |
| O | $CH_3$ | H | F | $CH_3$ | H | 2 or 1 |
| O | $CH_3$ | H | F | 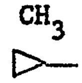 | H | 2 or 1 |
| O | H | Cl | F | 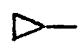 | H | 2 or 1 |
| O | H | F | F | 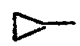 | H | 2 or 1 |

and the corresponding compounds where X is S.

In one preferred form of the invention, X is O. Compounds are also preferred where $R^1$ is methyl or methoxy and $R^2$ is methyl. Also preffered are compounds where Y is hydrogen or bromine and Z is hydrogen. In a further preferred form of the invention, $R^3$ is methyl. Preferably, n is 2.

The compounds of Formula I of the invention may be prepared from known starting materials, for example, by treating a phenylurea aldoxime or phenylurea ketoxime of Formula II

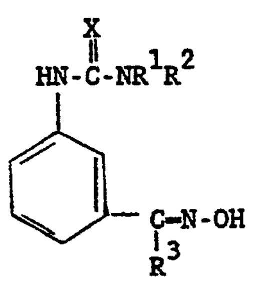

II

in which X, $R^1$ and $R^2$ and $R^3$ have the above meanings, and which are known from U.S. Patent No. 3,914,300, with dihydropyran or dihydrofuran at ambient temperatures of about 20°C in the prsence of acid and, preferably, an inert solvent, such as tetrahydrofuran, diethyl ether and the like.

Alternatively, the compounds of Formula I, including those wherein Y and Z are other than hydrogen, may also be prepared by nitration of the appropriate Y- and Z-substituted benzaldehyde, e.g. with $KNO_3$ and $H_2SO_4$, followed by addition of acidic methanol to form an acetal and reduction of the nitro group to an amino group with hydrogen in the presence of a catalyst e.g. Raney nickel. The resulting amine is treated successively with (a) $CXCl_2$ (eg $COCl_2$) in acetic acid, (b) an appropriate ammonia derivative, e.g., N-methoxy-N-methyl ammonium chloride in tetrahydrofuran and (c) hydroxylamine to give the corresponding Y- and Z-substituted phenylurea oxime. Treatment of this urea with 2,3-dihydrofuran or 3,4-dihydro-2H-pyran at ambient temperatures of about 20°C in the presence of acid, and optionally an inert solvent, such as tetrahydrofuran and the like gives the desired compound of Formula I.

Therefore according to a further aspect of the present invention, we provide a process for the preparation of a compound of Formula I as defined above which comprises reacting a compound of Formula III

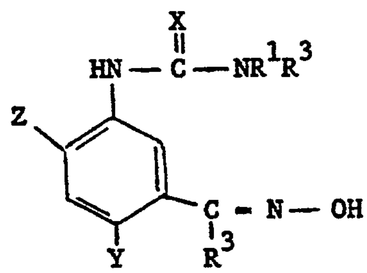

III

where $R^1$, $R^2$, $R^3$, X, Y and Z are as defined above, with dihydropyran or dihydrofuran.

The products may be recovered by conventional techniques such as precipitation, crystallization, filtration, solvent extraction, chromatography and the like.

For application, a compound of Formula I ordinarily is applied most effectively by formulating it with a suitable inert carrier or surface-active agent, or both. The invention, therefore, also includes compositions suitable for combatting unwanted plants, such compositions comprising an inert carrier or surface-active agent, or both, and as active ingredient at least one compound of Formula I.

The term "carrier" as used herein means an inert solid or liquid material, which may be inorganic or organic and of synthetic or natural origin, with which the active compound is mixed or formulated to facilitate its application to the plant, seed, soil or other object to be treated, or its storage, transport and/or handling. Any of the materials customarily employed in formulating pesticides, herbicides, or fungicides --i.e., horticulturally acceptable carriers -- are suitable.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements such as, for example, carbon and sulphur; natural and synthetic resins such as, for example, coumarone resins, polyvinyl chloride, and styrene polymers and copolymers; bitumen; waxes such as, for example, beeswax, paraffin wax, and chlorinated mineral waxes; solid fertilizers, for example, superphosphates; and ground, naturally-occurring, fibrous materials, such as ground corncobs.

Example of suitable liquid carriers are water, alcohols such as isopropyl alcohol and glycols; ketones such as acetone, methyl ethyl ketone, methyl isobutyl ketone and cyclohexanone; ethers such as cellosolves; aromatic hydrocarbons such as benzene, toluene and xylene; petroleum fractions such as kerosene and light mineral oils; chlorinated hydrocarbons such as carbon tetrachloride, perchloroethylene and trichloromethane. Also suitable are liquefied, normally vaporous and gaseous compounds. Mixtures of different liquids are often suitable.

The surface-active agent may be an emulsifying agent or a dispersing agent or a wetting agent; it may be nonionic or ionic. Any of the surface-active agents usually applied in formulating herbicides or insecticides may be used. Examples of suitable surface-active agents are the sodium and calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products, alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate, and polymers of ethylene oxide and copolymers of ethylene oxide and propylene oxides.

The compositions of the invention may be prepared as wettable powders, dusts, granules, solutions, emulsifiable concentrates, emulsions, suspension concentrates and aerosols. Wettable powders are usually compounded to contain 25 to 75% by weight of active compound and usually contain, in addition to the solid carrier, 3-10% by weight of a dispersing agent, 2-15% of a surface-active agent and, where necessary, 0-10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 0.5-10% by weight of the active compound. Granules are usually prepared to have a size between 10 and 100 BS mesh (1.676-0.152 mm), and may be manufactured by agglomeration or impregnation techniques. Generally, granules will contain 0.5-25% by weight of the active compound, 0.-1% by weight of additives such as stabilizers, slow release modifiers and binding agents. Emulsifiable concentrates usually contain, in addition to the solvent and, when necessary, cosolvent, 10-50% weight per volume of the active compound, 2-20% weight per volume emulsifiers and 0-20% weight per volume of appropriate additives such as stabilizers, pentrants and corrosion inhibitors. Suspension concentrates are compounded so as to obtain a stable, non-sedimenting, flowable product and usually contain 10-75% weight of the active compound, 0.5-5% weight of dispersing agents, 1-5% of surface-active agent, 0.1-10% weight of suspending agents, such as defoamers, corrosion inhibitors, stabilizers, penetrants and stickers, and as carrier, water or an organic liquid in which the active compound is substantially insoluble; certain organic solids or inorganic salts may be dissolved in the carrier to assist in preventing sedimentation or as antifreeze agents for water.

Of particular interest in current practice are water-dispersible granular formulations. There are in the form of dry, hard granules that are essentially dust-free, and are resistant to attrition on handling, thus minimizing the formation of dust. On contact with water, the granules readily disintegrate to form stable suspensions of the particles of active material. Such formulations contain 90% or (up to 95%) more by weight of finely divided active material, 3-7% by weight of a blend of surfactants, which act as wetting, dispersing, suspending and binding agents, and may contain up to 3% by weight of a finely divided carrier, which acts as a resuspending agent.

Aqueous dispersions and emulsions, for example, compositions obtained by diluting a wettable powder or a concentrate according to the invention with water, also lie within the scope of the present invention. The said emulsions may be of the water-in-oil or of the oil-in-water type, and may have thick, mayonnaise-like consistency.

It is evident from the foregoing that this invention contemplates compositions containing as little as about 0.5% by weight to as much as about 95% by weight of a compound of Formula I as the active ingredient.

The compositions of the invention may also contain other ingredients, for example, other compounds possessing pesticidal, especially insecticidal, acaricidal, herbicidal or fungicidal properties, as are appropriate to the intended purpose.

Protection of a locus or area from undesirable plants is effected by applying a compound of Formula I, ordinarily in a composition of one of the aforementioned types, to soil in which the seeds of the unwanted plants are present, or to the foliage of the unwanted plants, particularly broadleaf weeds. In some cases, the compounds of Formula I are selective to broadleaf crops, e.g. cotton, soybean and the like. The active compound, of course, is applied in an amount sufficient to exert the desired action.

The amount of the compound of the invention to be us ed in combatting undesired plants will naturally depend on the condition of the plants, the degree of activity desired, the formulation used, the mode of application, the climate, the season of the year, and other variables. Recommendations as to precise amounts are, therefore, not possible. In general, howevr, application to the locus to be protected of from 0.1 to 10.0 kg per hectare of the compound of Formula I will be satisfactory.

## Examples

The present invention is illustrated by the following examples, which should not be regarded as limiting the invention in any way. The products, including intermediates, were identified by elemental, infrared and nuclear magnetic resonance (NMR) spectral analyses as necessary.

### Example 1: Compound of Formula Ia

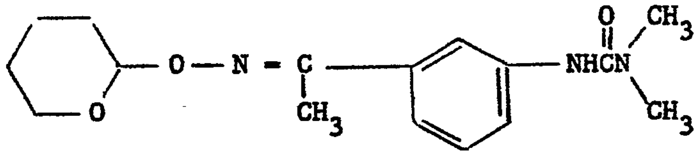

Ia

To a slurry of 6.0 g of the corresponding ketoxime urea (disclosed in U.S. Patent No. 3,914,300) in 30 ml of tetrahydrofuran was added 9.1 g of dihydropyran, and five drops of concentrated hydrochloric acid. The resulting solution was allowed to stir at room temperature for seventy-two hours. The reaction mixture was diluted with ether, and the solid was collected by suction-filtration and dried under vacuum to give 6.9 g of white solid, m.p.: 146-148°C.

Example 2: Compound of Formula Ib

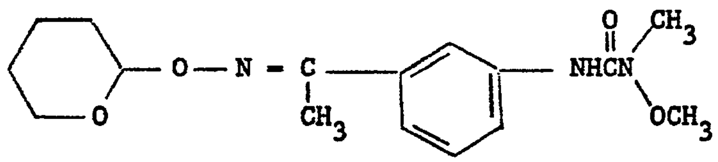

Ib

The above compound was prepared as an amber oil by a process similar to Example 1 above, by treating the corresponding ketoxime urea disclosed in U.S. Patent No. 3,914,300 with dihydropyran in tetrahydrofuran containing a catalytic amount of concentrated hydrochloric acid.

Example 3: Compound of Formula Ic

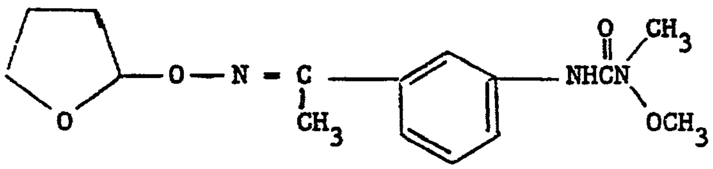

Ic

To a solution of 2.0 g of the corresponding ketoxime urea (disclosed in U.S. Patent No. 3,914,300) in 25 ml of tetrahydrofuran was added 2.2 g of dihydrofuran and five drops of concentrated hydrochloric acid. The solution was allowed to stir at room temperature for seventy-two hours. The reaction mixture was diluted with 100 ml of ether and washed with 10% aqueous sodium hydroxide (3 x 100 ml). The organic phase was washed with brine, dried over anhydrous magnesium sulfate, suction-filtered, concentrated and adsorbed on silica gel. Purification by chromatography with the solvent mixture (by volume):hexane (80), ethyl acetate (16), tetrahydrofuran (4), gave 1.2 g of the desired product as a light yellow syrup.

Example 4: Compound of Formula Id

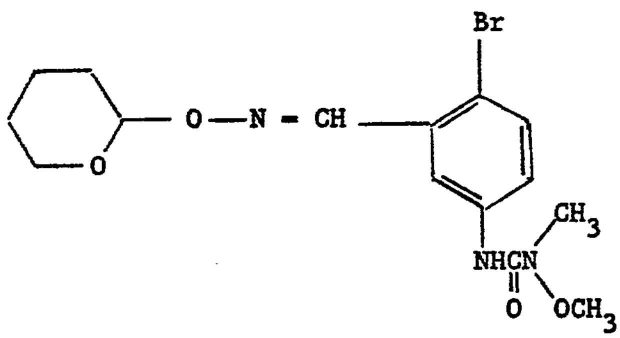

Id

(a) 2-Bromo-5-nitrobenzaldehyde

Potassium nitrate, 54.4 g, was added portionwise to a stirred and chilled (0-5°C) solution containing 99.9 g of 2-bromo-benzaldehyde in 500 ml of concentrated sulfuric acid. After 0.5 hour, the mixture was poured over ice-water, and the product was filtered, washed with water and recrystallized from ether-hexane to give 95.6 g of the desired benzaldehyde, m.p.: 99-100°C.

(b) 2-Bromo-5-nitrobenzaldehyde dimethyl acetal

A solution containing 94.1 g of the benzaldehyde prepared in (a) above and 1.0 g of hydrogen chloride in 1000 ml of methanol was allowed to stand at ambient temperature for one week. An off-white solid that had formed was removed by filtration. The filtrate was concentrated to a volume of 75 ml and, after cooling, a solid was filtered. The combined solids amounted to 92.2 g, m.p.: 68-71°C.

(c) 2-Bromo-5-aminobenzaldehyde dimethyl acetal

A mixture containing 92.2 g of the acetal prepared in (b) above and 2.5 g of Raney nickel in 1000 ml of methanol was heated to reflux while 60 ml of 85% hydrazine hydrate was added dropwise over a period of 8 hours. The reaction mixture was filtered and the filtrate was concentrated to a volume of 200 ml, mixed with ether, washed well with water, dried, and concentrated to give 72.6 g of the desired compound as an amber oil. GLC indicated a purity of 90%

(d) N'-(4-Bromo-3-((hydroxyimino)methyl)phenyl)-N-methoxy-N-methyl urea

A solution containing 12.3 g of 2-bromo-5-aminobenzaldehyde dimethyl acetal prepared in (c) above and 45 g of phosgene in 300 ml of ethyl acetate was refluxed for 4 hours and concentrated under reduced pressure. A solution of the residual oil in 25 ml of tetrahydrofuran was added with stirring to a chilled (5-10°C) solution containing 7.5 g of O,N-dimethylhydroxylamine hydrochloride and 4.5 g of sodium carbonate in 50 ml of water. After one hour, the reaction mixture was acidified with 30 ml of concentrated hydrochloric acid and filtered. Recrystallization of the filter cake from methanol gave 11.2 g of white solid; m.p.: 222-225°C. A slurry of 5.74 g of that compound in 100 ml of methanol was successively treated with a solution of sodium acetate (2 g) in 25 ml of water and hydroxylamine hydrochloride (1.5 g) in 20 ml of water. After one hour, the stirred reaction mixture was poured into 250 ml of water and filtered. The filter cake contained two major compounds (by thin layer chromatography) which were separated by silica chromatography. The compound that emerged from the column was recrystallized from ether-hexane to give 2.3 g of the intermediate urea 4(d), m.p.: 150-152°C.

(e) Compound of Formula Id

A solution of 0.35 g of the aldoxime urea prepared in (d) above in 50 ml of dihydropyran containing 5 drops of concentrated hydrochloric acid was heated on a steam bath for one hour. the cooled reaction mixture was diluted with ether, and washed with 10% aqueous sodium hydroxide (3 x 100 ml). The organic phase was dried ($MgSO_4$), concentrated, and purified by flash chromatography with methylene chloride as eluent. Yield, 0.1 g of the desired product of Formula Id as a yellow syrup.

Example 5: Compound of Formula Ie

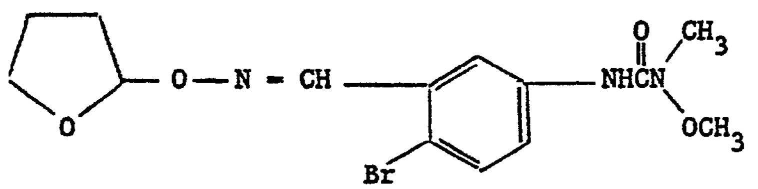

Ie

A solution of 0.35 g of the aldoxime urea of Example 4(d) above in 50 ml of dihydrofuran containing 5 drops of concentrated hydrochloric acid was heated to reflux for one hour. The cooled reaction mixture was diluted with 100 ml of ether and washed with 10% aqueous sodium hydroxide (3 x 100 ml). The organic phase was then washed with brine, dried over anhydrous magnesium sulfate, filtered, concentrated under reduced pressure, and purfied by flash column chromatography with silica gel for support and the following solvent mixture (by volume) as eluent:hexane (66), ethyl acetate (30) tetrahydrofuran (4), to give 0.3 g of the desired product as a light yellow syrup, which crystallized on standing; m.p.: 95-98°C.

Examples 6-21

Following procedures similar to those described in Examples 1-5 above, the following additional representative species are prepared as set forth in Table 1 below.

Table 1

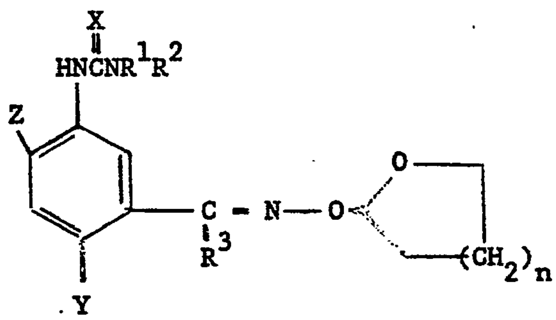

(I)

| Example | $R^1$ | Y | Z | $R^2$ | $R^3$ | n |
|---|---|---|---|---|---|---|
| 6 | $CH_3$ | H | H | $CH_3$ | $OCH_3$ | 2 |
| 7 | $CH_3$ | H | F | $CH_3$ | $CH_3$ | 2 |
| 8 | H | $CH_3$ | H | $CH_3$ | $OCH_3$ | 2 |
| 9 | H | $CH_3$ | F | $CH_3$ | $CH_3$ | 2 |
| 10 | H | Cl | H | $CH_3$ | $OCH_3$ | 2 |
| 11 | H | Cl | F | $CH_3$ | $OCH_3$ | 2 |
| 12 | H | F | H | $CH_3$ | $OCH_3$ | 2 |
| 13 | $CH_3$ | F | H | $CH_3$ | $OCH_3$ | 2 |
| 14 | $CH_3$ | F | H | $CH_3$ | $CH_3$ | 2 |
| 15 | $CH_3$ | H | H | $CH_3$ | H | 2 |
| 16 | $CH_3$ | H | F | $CH_3$ | H | 2 |
| 17 | $CH_3$ | H | F | 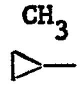 | H | 2 |
| 18 | H | Cl | F | 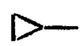 | H | 2 |
| 19 | H | F | F | 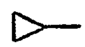 | H | 2 |

Example 20: Activity with Respect to Plants

In the following tests, the species of plants that were tested were:

| | Abbreviation |
|---|---|
| Barnyardgrass (watergrass) – Echinochloa crus-galli | BYGR |
| Downy brome – Bromus tectorum | DOBR |
| Yellow foxtail – Setaria glauca | YEFT |
| Sicklepod – Cassia obtusifolia | SIPO |
| Velvetleaf – Abutilon theophrasti | VELE |
| Garden cress – Lepidium sativum | GACR |
| Johnsongrass – Sorghum halepense | JOGR |
| Morningglory – Ipomoea sp. | MOGL |
| Field bindweed – Convolvulus arvensis | FIBW |
| Nightshade – Solanum sp. | NISH |
| Blackgrass – Alopecurus myosuroides | BLGR |
| Yellow millet – Panicum miliaceum | YEMI |
| Large crabgrass – Digitaria sanguinalis | LACG |
| Redroot pigweed – Amaranthus retroflexus | RRPW |
| Hemp sesbania – Sesbania exaltata | HESE |
| Prickly sida – Sida spinosa | PRSI |

Test Procedures

The preemergence (soil) herbicidal activity of compounds of Formula I was evaluated by planting seeds of downy brome, johnsongrass, yellow foxtail, barnyardgrass, yellow millet, blackgrass, hemp sesbania, velvetleaf, morningglory, prickly sida, sicklepod and garden cress in test tubes, nominally measuring 25 x 200 millimeters, filled about three-quarters full of untreated soil, in each case covered on top with about 2.5 cubic centimeters of soil treated with 0.1 milligram of the test compound, to give a dosage of 2.24 kg of test compound per hectare (2.0 pounds per acre). The seeds were planted on top of the treated soil and covered with about 1.5 cubic centimeters of untreated soil. The planted soil was held under a controlled regimen of temperature, moisture, and light. At 10 days, the amounts of germination and growth in each tube were evaluated on a 0 to 9 scale, the numeric ratings having the following meanings:

| Rating | Meaning |
|---|---|
| 9 | No living tissue |
| 8 | Plant severely damaged and expected to live |
| 7 | Plant badly damaged, but expected to live |
| 6 | Moderate damage, but complete recovery expected |
| 5 | Intermediate damage (probably unacceptable for crop plants) |
| 3–4 | Observable damage |
| 1–2 | Plant slightly affected, possibly by the chemical, possibly due biological variability |
| 0 | No visible effect |

The postemergence (foliar) herbicidal activity of compounds of Formula I was evaluted by spraying 9-day-old large crabgrass plants, 9-day-old pigweed plants, 6-day-old johnsongrass plants, 9-day-old velvetleaf palnts, 8-day-old yellow foxtail plants, 9-day-old sicklepod plants, 5-day-old morningglory plants, 5-day-old barnyardgrass plants, 6-day-old yellow millet plants, 9-day-old nightshade plants, 9-day-old prickly sida plants and 7-day-old field bindweed plants to runoff with 2.4 milliliters of a liquid formulation containing 0.5 milligram of the test compound (1.12 kg of the compound per hectare, one pound of the test compound per acre). The sprayed plants were held under a controlled regimen of temperature,

moisture and light for 7 to 8 days, when the effect of the test compound was evaluated visually, the results being rated on the 0 to 9 scale described above.

Results of the preemergence and postemergence herbicidal activity tests are set forth in Tables I and II.

Table I. Preemergence Herbicidal Activity

| Compound No. | DOBR | JOGR | YEFT | BYGR | YEMI | BLGR | HESE | VELE | MOGL | PRSI | SIPO | GACR |
|---|---|---|---|---|---|---|---|---|---|---|---|---|
| 1 | 4 | 4 | 0 | 3 | 0 | 3 | 6 | 6 | 7 | 0 | 8 | 5 |
| 2 | 4 | 0 | 3 | 5 | 0 | 5 | 7 | 3 | 9 | 7 | 8 | 9 |
| 3 | 9 | 7 | 5 | 9 | 7 | 5 | 9 | 7 | 7 | 5 | 9 | 8 |
| 4 | 0 | 0 | 0 | 0 | 0 | 0 | 0 | 0 | 0 | 0 | 0 | 5 |
| 5 | 0 | 3 | 0 | 7 | 0 | 3 | 7 | 4 | 4 | 4 | 0 | 7 |

Table II. Postemergence Herbicidal Activity

| Compound No. | LACG | JOGR | YEFT | BYGR | YEMI | RRPW | NISH | VELE | MOGL | PRSI | SIPO | FIBW |
|---|---|---|---|---|---|---|---|---|---|---|---|---|
| 1 | 2 | 3 | 3 | 2 | 2 | 7 | 4 | 5 | 7 | 3 | 6 | 4 |
| 2 | 0 | 2 | 4 | 2 | 3 | 5 | 5 | 4 | 7 | 5 | 5 | 6 |
| 3 | 3 | 4 | 3 | 3 | 4 | 4 | 8 | 6 | 6 | 5 | 7 | 5 |
| 4 | 5 | 5 | 8 | 4 | 0 | 5 | 4 | 0 | 3 | 0 | 4 | 9 |
| 5 | 4 | 6 | 8 | 4 | 5 | 6 | 8 | 3 | 7 | 3 | 5 | 8 |

**Claims for the contracting states: BE, CH, DE, FR, GB, GR, IT, LI, LU, NL, SE**

1. A compound of the Formula I

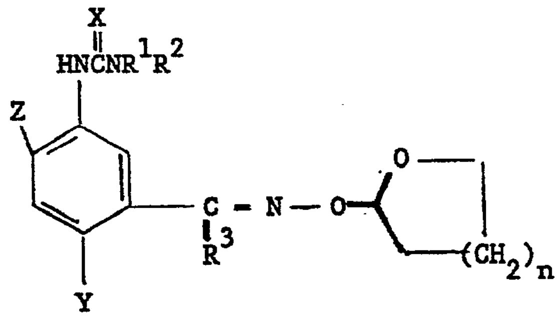

(I)

wherein $R^1$ and $R^2$ each independently is a hydrogen atom, cyclopropyl, or alkyl or alkoxy containing 1 to 5 carbon atoms; $R^3$ is hydrogen or alkyl or alkoxy containing 1 to 3 carbon atoms; X is 0 or S; Y is hydrogen, alkyl containing 1 to 4 carbon atoms, fluorine, chlorine or bromine; Z is hydrogen or fluorine; and n is 1 or 2.

2. A compound according to claim 1 wherein X is 0.

3. A compound according to claim 1 or 2 wherein $R^1$ is methyl or methoxy and $R^2$ is methyl.

4. A compound according to claim 1, 2 or 3 wherein Y is hydrogen or bromine and Z is hydrogen.

5. A compound according to any one of claims 1 to 4 wherein $R^3$ is methyl.

6. A compound according to anyone of claims 1 to 5 wherein n is 2.

7. A process for the preparation of a compound of Formula I as defined in claim 1 which comprises reacting a compound of Formula III

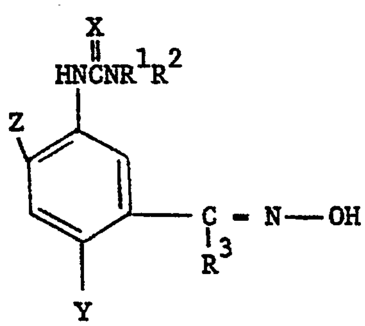

where $R^1$, $R^2$, $R^3$, X, Y and Z are as defined in claim 1, with dihydropyran or dihydrofuran.

8. A process according to claim 7 wherein the reaction is carried out at ambient temperature in an inert solvent in the presence of acid.

9. A compound of Formula I when obtained by the process of claim 7 or 8.

10. A herbicidal composition which comprises a herbicidally effective amount of a compound according to any one of claims 1 to 6 or 9 and at least one surface-active agent and/or carrier.

11. A method for combatting unwanted plant growth at a locus which comprises applying to the locus a herbicidally effective amount of a compound according to anyone of claims 1 to 6 or 9 or a composition according to claim 10.

12. A method according to claim 11 wherein the combatting of unwanted plant growth is selective control of weeds in cotton.

**Claims for the contracting state: AT**

1. A herbicidal composition, which comprises a herbicidally effective amount of a compound of the Formula I

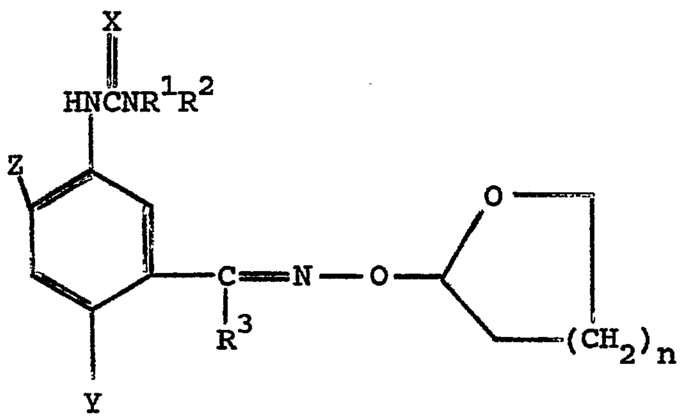

(I)

wherein $R^1$ and $R^2$ each independently is a hydrogen atom, cyclopropyl, or alkyl or alkoxy containing 1 to 5 carbon atoms; $R^3$ is hydrogen or alkyl or alkoxy containing 1 to 3 carbon atoms; X is O or S; Y is hydrogen, alkyl containing 1 to 4 carbon atoms, fluorine, chlorine or bromine; Z is hydrogen or fluorine; and n is 1 or 2; and at least one surface-active agent and/or carrier.

2. A composition according to claim 1, wherein in the Formula I X is O.

3. A composition according to claim 1 or 2, wherein in the Formula I $R^1$ is methyl or methoxy and $R^2$ is methyl.

4. A composition according to claim 1, 2, or 3, wherein in the Formula I Y is hydrogen or bromine and Z is hydrogen.

5. A composition according to any one of claims 1 to 4, wherein in the Formula I $R^3$ is methyl.

6. A composition according to any one of claims 1 to 5, wherein in the Formula I n is 2.

7. A process for the preparation of a compound of Formula I as defined in claim 1, which comprises reacting a compound of Formula III

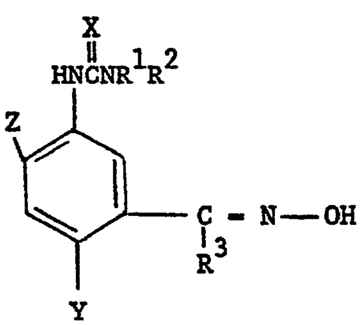

where $R^1$, $R^2$, $R^3$, X, Y and Z are as defined in claim 1, with dihydropyran or dihydrofuran.

8. A process according to claim 7, wherein the reaction is carried out at ambient temperature in an inert solvent in the presence of acid.

9. A method for combating unwanted plant growth at a locus, which comprises applying to the locus a herbicidally effective amount of a compound as defined in any one of claims 1 to 6, or a composition as claimed in any one of claims 1 to 6.

10. A method according to claim 9, wherein the combating of unwanted plant growth is the selective control of weeds in cotton.

**Claims for the contracting state: ES**

1. A process for the preparation of a compound of the Formula I

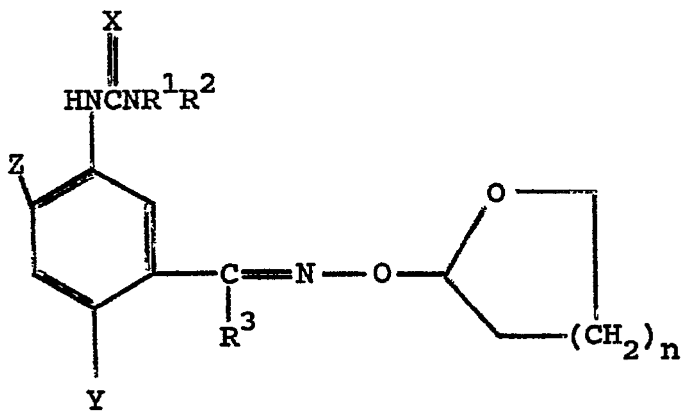

(I)

wherein $R^1$ and $R^2$ each independently is a hydrogen atom, cyclopropyl, or alkyl or alkoxy containing 1 to 5 carbon atoms; $R^3$ is hydrogen or alkyl or alkoxy containing 1 to 3 carbon atoms; X is O or S; Y is hydrogen, alkyl containing 1 to 4 carbon atoms, fluorine, chlorine or bromine; Z is hydrogen or fluorine; and n is 1 or 2; which comprises reacting a compound of Formula III

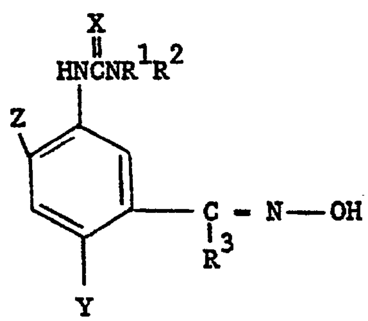

where $R^1$, $R^2$, $R^3$, X, Y and Z are as defined above, with dihydropyran or dihydrofuran.

2. A process according to claim 1, wherein the reaction is carried out at ambient temperature in an inert solvent in the presence of acid.

3. A process according to claim 1 or 2, wherein in the Formulae I and III X is O.

4. A process according to any one of claims 1 to 3, wherein in the Formulae I and III $R^1$ is methyl or methoxy and $R^2$ is methyl.

5. A compound according to any one of claims 1 to 4, wherein in the Formulae I and III Y is hydrogen or bromine and Z is hydrogen.

6. A compound according to any one of claims 1 to 5 wherein in the Formulae I and III $R^3$ is methyl.

7. A compound according to anyone of claims 1 to 6, wherein in the Formula I n is 2.

8. A method for combatting unwanted plant growth at a locus which comprises applying to the locus a herbicidally effective amount of a compound of Formula I as defined in anyone of claims 1 and 3 to 6 or a herbicidal composition which comprises a herbicidally effective amount of a compound of Formula I and at least one surface active agent and/or carrier.

9. A method according to claim 8, wherein the combatting of unwanted plant growth is selective control of weeds in cotton.

**Revendications pour les états contractants: BE, CH, DE, FR, GB, GR, IT, LU, NL, SE**

1. Un composé de formule I:

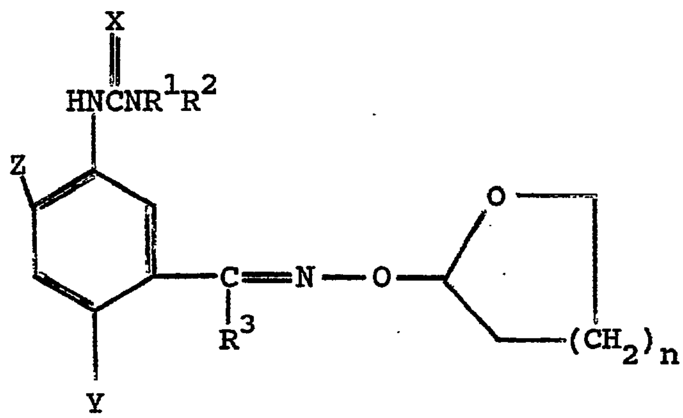

(I)

dans lequel $R^1$ et $R^2$ représentent chacun indépendamment un atome d'hydrogène, un radical cyclopropyle, ou alkyle ou bien alcoxy renfermant de 1 à 5 atomes de carbone; $R^3$ est de l'hydrogène ou bien un radical alkyle ou alcoxy renfermant 1 à 3 atomes de carbone; X est O ou S; Y est de 1l'hydrogène, un radical alkyle contenant de 1 à 4 atomes de carbone, du fluor, du chlore ou du brome; Z est de l'hydrogène ou du fluor; et n est 1 ou 2.

2. Un composé selon la revendication 1, dans lequel X est O.

3. Un composé selon la revendication 1 ou 2, dans lequel $R^1$ est du méthyle ou du méthoxy et $R^2$ est du méthyle.

4. Un composé selon la revendication 1, 2 ou 3, dans lequel Y est de l'hydrogène ou du brome et Z est de l'hydrogène.

5. Un composé selon l'une quelconque des revendications 1 à 4, dans lequel $R^3$ est du méthyle.

6. Un composé selon l'une quelconque des revendications 1 à 5, dans lequel n est 2.

7. Un procédé pour la préparation d'un composé de formule I tel que défini dans la revendication 1, dans lequel on fait réagir un composé de formule III

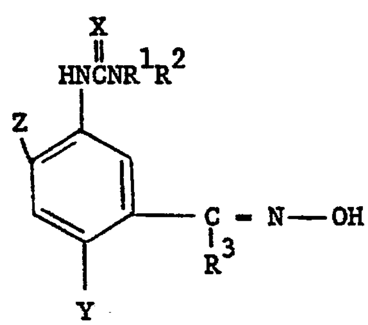

dans lequel $R^1$, $R^2$, $R^3$, X, Y et Z sont tels que définis dans la revendication 1, avec du dihydropyrane ou du dihydrofurane.

8. Un procédé selon la revendication 7, dans lequel la réaction est mise en œuvre à la température ambiante dans un solvant inerte en présence d'un acide.

9. Un composé de la formule I, lorsqu'il est obtenu par le procédé de la revendication 7 ou 8.

10. Un composé herbicide, qui comprend une quantité efficace du point de vue herbicide d'un composé selon l'une quelconque des revendications 1 à 6 ou 9 et au moins un agent tensio-actif et/ou un support.

11. Une méthode pour combattre la croissance des plantes indésirables en un lieu, qui comprend l'application sur le lieu d'une quantité efficace du point de vue herbicide d'un composé selon l'une quelconque des revendications 1 à 6 ou 9, ou bien d'une composition selon la revendication 10.

12. Une méthode selon la revendication 1, dans laquelle le combat contre la croissance des plantes indésirables est un contrôle sélectif des mauvaises herbes dans le coton.

**Revendications pour l'état contractant: AT**

1. Une composition herbicide qui comprend une quantité efficace du point de vue herbicide d'un composé de formule I

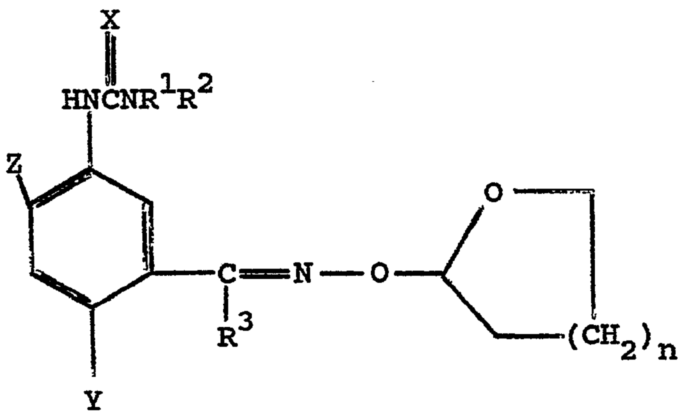

(I)

dans laquelle $R^1$ et $R^2$ représentent chacun indépendamment un atome d'hydrogène, un radical cyclopropyle, ou alkyle ou bien alcoxy renfermant de 1 à 5 atomes de carbone; $R^3$ est de l'hydrogène ou bien un radical alkyle ou alcoxy renfermant 1 à 3 atomes de carbone; X est O ou S; Y est de l'hydrogène, un radical alkyle contenant de 1 à 4 atomes de carbone, du fluor, du chlore ou du brome; Z est de l'hydrogène ou du fluor; et n est 1 ou 2; et au moins un agent tensio-actif et/ou un support.

2. Une composition selon la revendication 1, dans laquelle X est O.

3. Une composition selon la revendication 1 ou 2, dans laquelle dans la formule I $R^1$ est du méthyle ou du méthoxy et $R^2$ est du méthyle.

4. Une composition selon la revendication 1, 2 ou 3, dans laquelle dans la formule I Y est de l'hydrogène ou du brome et Z est de l'hydrogène.

5. Une composition selon l'une quelconque des revendications 1 à 4, dans laquelle dans la formule I $R^3$ est du méthyle.

6. Une composition selon l'une quelconque des revendications 1 à 5, dans laquelle dans la formule I n est 2.

7. Un procédé pour la préparation d'un composé de formule I tel que défini dans la revendication 1, dans lequel on fait réagir un composé de formule III

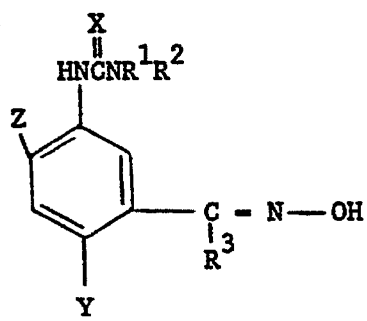

dans lequel $R^1$, $R^2$, $R^3$, X, Y et Z sont tels que définis dans la revendication 1, avec du dihydropyrane ou du dihydrofurane.

8. Un procédé selon la revendication 7, dans lequel la réaction est mise en œuvre à la température ambiante dans un solvant inerte en présence d'un acide.

9. Une méthode pour combattre la croissance de plantes indésirables en un lieu, qui consiste à appliquer sur le lieu une quantité effective du point de vue herbicide d'un composé tel que défini dans l'une quelconque des revendications 1 à 6, ou bien une composition telle que revendiquée dans l'une quelconque des revendications 1 à 6.

10. Une méthode selon la revendication 9, dans laquelle le combat contre la croissance des plantes indésirables est le contrôle sélectif des mauvaises herbes dans le coton.

**Revendications pour l'état contractant: ES**

1. Un procédé de préparation d'un composé selon la formule I ·

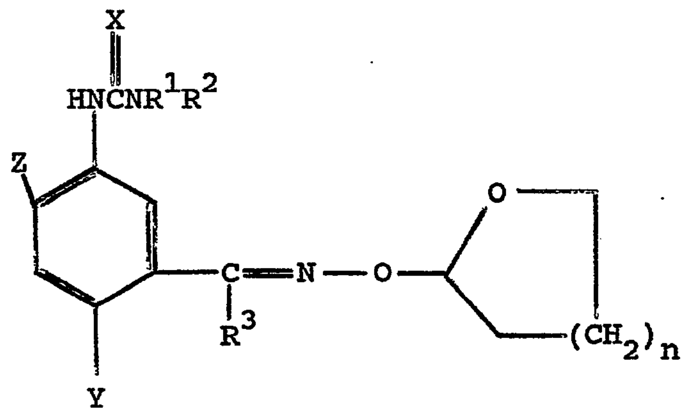

(I)

dans lequel $R^1$ et $R^2$ représentent chacun indépendamment un atome d'hydrogène, un radical cyclopropyl, ou alkyl ou bien alcoxy renfermant de 1 à 5 atomes de carbone; $R^3$ est de l'hydrogène ou bien un radical alkyle ou alcoxy renfermant 1 à 3 atomes de carbone; X est O ou S; Y est de l'hydrogène, un radical alkyle contenant de 1 à 4 atomes de carbone, du fluor, du chlore ou du brome; Z est de l'hydrogène ou du fluor; et n est 1 ou 2; dans lequel on fait réagir un composé de formule III

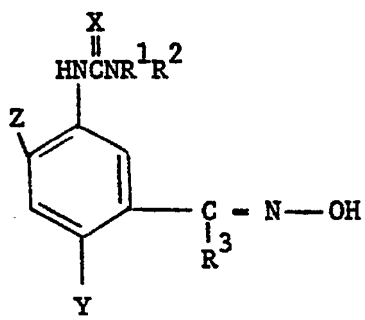

dans laquelle $R^1$, $R^2$, $R^3$, X, Y et Z sont tels que définis ci-dessus avec du dihydropyrane ou du dihydrofurane.

2. Un procédé selon la revendication 1, dans lequel la réaction est mise en œuvre à la température ambiante dans un solvant inerte en présence d'un acide.

3. Un procédé selon la revendication 1 ou 2, dans lequel dans les formules I et III, X est O.

4. Un procédé selon l'une quelconque des revendications 1 à 3, dans lequel dans les formules I et III, $R^1$ est du méthyle ou du méthoxy et $R^2$ est du méthyl.

5. Un composé selon l'une quelconque des revendications 1 à 4, dans lequel dans les formules I et III, Y est de l'hydrogène ou du brome et Z est de l'hydrogène.

6. Un composé selon l'une quelconque des revendications 1 à 5, dans lequel dans les formules I et III, $R^3$ est du méthyle.

7. Un composé selon l'une quelconque des revendications 1 à 6, dans lequel dans la formule I, n est 2.

8. Une méthode pour combattre la croissance des plantes indésirables en un lieu, dans laquelle on applique sur le lieu une quantité efficace du point de vue herbicide un composé de formule I tel que défini dans l'une quelconque des revendications 1 et 3 à 6 ou une composition herbicide qui comprend une quantité efficace du point de vue herbicide d'un composé de formule I et au moins un agent tension-actif et/ou un support.

9. Une méthode selon la revendication 8, dans laquelle le combat contre la croissance des plantes indésirables est le contrôle sélectif des mauvaises herbes dans le coton.

**Patentansprüche für die Vertragsstaaten: BE, CH, DE, FR, GB, GR, IT, LU, NL, SE**

1. Verbindung der Formel I

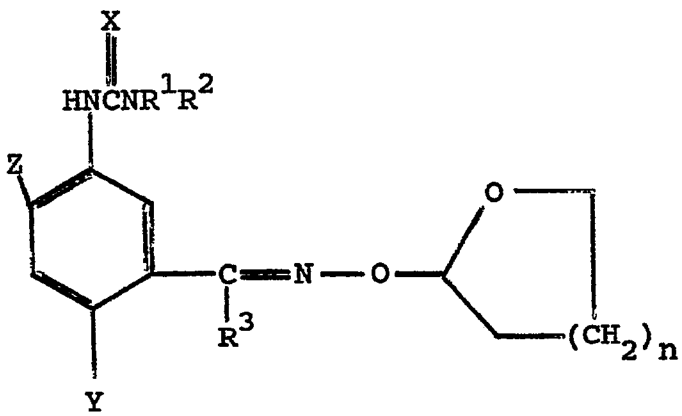

(I)

worin $R^1$ und $R^2$ jeweils unabhängig voneinander ein Wasserstoffatom, Cyclopropyl oder Alkyl oder Alkoxy mit 1 bis 5 Kohlenstoffatomen darstellen; $R^3$ Wasserstoff oder Alkyl oder Alkoxy mit 1 bis 3 Kohlenstoffatomen ist; X für O oder S steht; Y Wasserstoff, Alkyl mit 1 bis 4 Kohlenstoffatomen, Fluor, Chlor oder Brom darstellt; Z Wasserstoff oder Fluor ist; und n den Wert 1 oder 2 besitzt.

2. Verbindung nach Anspruch 1, worin X für O steht.

3. Verbindung nach Anspruch 1 oder 2, worin $R^1$ Methyl oder Methoxy darstellt und $R^2$ Methyl ist.

4. Verbindung nach Anspruch 1, 2 oder 3, worin Y für Wasserstoff oder Brom steht und Z Wasserstoff ist.

5. Verbindung nach einem der Ansprüche 1 bis 4, worin $R^3$ Methyl ist.

6. Verbindung nach einem der Ansprüche 1 bis 5, worin n den Wert 2 besitzt.

7. Verfahren zur Herstellung einer Verbindung der Formel I, wie in Anspruch 1 definiert, welches das Umsetzen einer Verbindung der Formel II'

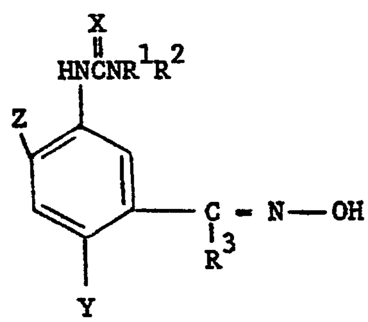

worin $R^1$, $R^2$, $R^3$, X, Y und Z wie in Anspruch 1 definiert sind, mit Dihydropyran oder Dihydrofuran, umfaßt.

8. Verfahren nach Anspruch 7, worin die Umsetzung bei Umgebungstemperatur in einem inerten Lösungsmittel in Gegenwart von Säure ausgeführt wird.

9. Durch das Verfahren von Anspruch 7 oder Anspruch 8 erhaltene Verbindung der Formel I.

10. Herbizide Zusammensetzung, welche eine herbizid wirksame Menge einer Verbindung nach einem der Ansprüche 1 bis 6 oder 9 und mindestens ein oberflächenaktives Mittel und/oder einen Träger umfaßt.

11. Verfahren zur Bekämpfung von unerwünschtem Pflanzenwuchs an einem Ort, umfassend Aufbringen einer herbizid wirksamen Menge einer Verbindung nach einem der Ansprüche 1 bis 6 oder 9 oder einer Zusammensetzung nach Anspruch 10 auf den Ort.

12. Verfahren nach Anspruch 11, worin die Bekämpfung von unerwünschtem Pflanzenwuchs eine selektive Bekämpfung von Unkräutern in Baumwolle ist.

**Patentansprüche für den Vertragsstaat: AT**

1. Herbizide Zusammensetzung, welche eine herbizide wirksame Menge einer Verbindung der Formel I

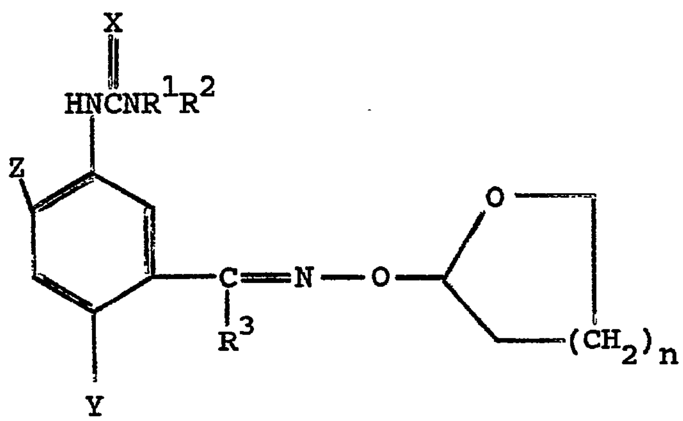

(I)

enthält, worin $R^1$ und $R^2$ jeweils unabhängig voneinander ein Wasserstoffatom, Cyclopropyl oder Alkyl oder Alkoxy mit 1 bis 5 Kohlenstoffatomen sind; $R^3$ Wasserstoff oder Alkyl oder Alkoxy mit 1 bis 3 Kohlenstoffatomen ist; X für O oder S steht; Y Wasserstoff, Alkyl mit 1 bis 4 Kohlenstoffatomen, Fluor, Chlor oder Brom darstellt; Z Wasserstoff oder Fluor ist; und n den Wert 1 oder 2 besitzt; und mindestens ein oberflächenaktives Mittel und/oder einen Träger umfaßt.

2. Zusammensetzung nach Anspruch 1, worin in der Formel I X für O steht.

3. Zusammensetzung nach Anspruch 1 oder 2, worin in der Formel I $R^1$ Methyl oder Methoxy darstelllt und $R^2$ Methyl ist.

4. Zusammensetzung nach Anspruch 1, 2 oder 3, worin in der Formel I Y für Wasserstoff oder Brom steht und Z Wasserstoff ist.

5. Zusammensetzung nach einem der Ansprüche 1 bis 4, worin in der Formel I $R^3$ für Methyl steht.

6. Zusammensetzung nach einem der Ansprüche 1 bis 5, worin in der Formel I n den Wert 2 besitzt.

7. Verfahren zur Herstellung einer Verbindung der Formel I, wie in Anspruch 1 definiert, welches das Umsetzen einer Verbindung der Formel III

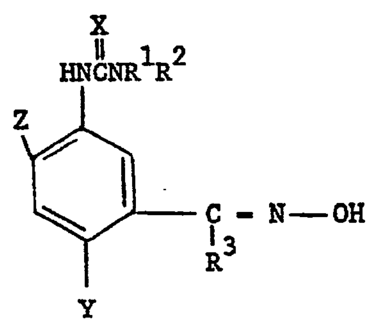

worin $R^1$, $R^2$, $R^3$, X, Y und Z wie in Anspruch 1 definiert sind, mit Dihydropyran oder Dihydrofuran umfaßt.

8. Verfahren nach Anspruch 7, worin die Umsetzung bei Umgebungstemperatur in einem inerten Lösungsmittel in Gegenwart von Säure ausgeführt wird.

9. Verfahren zum Bekämpfen von unerwünschtem Pflanzenwuchs an einem Ort, welches das Aufbringen einer herbizid wirksamen Menge einer Verbindung, wie in einem der Ansprüche 1 bis 6 auf den Ort umfaßt.

10. Verfahren nach Anspruch 9, worin das Bekämpfen von unerwünschtem Pflanzenwuchs die selektive Bekämpfung von Unkräutern in Baumwolle ist.

**Patentansprüche für den Vertragsstaat: ES**

1. Verfahren zur Herstellung einer Verbindung der Formel I,

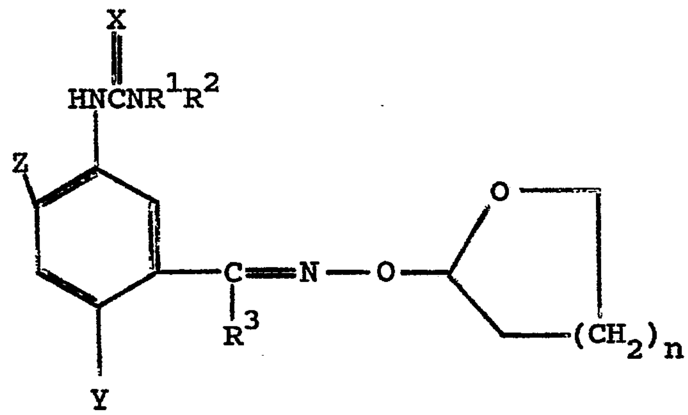

(I)

worin $R^1$ und $R^2$ jeweils unabhängig voneinander ein Wasserstoffatom, Cyclopropyl oder Alkyl oder Alkoxy mit 1 bis 5 Kohlenstoffatomen sind; $R^3$ Wasserstoff oder Alkyl oder Alkoxy mit 1 bis 3 Kohlenstoffatomen ist; X für O oder S steht; Y Wasserstoff, Alkyl mit 1 bis 4 Kohlenstoffatomen, Fluor, Chlor oder Brom darstellt; Z Wasserstoff oder Fluor; und n den Wert 1 oder 2 besitzt; welches das Umsetzen einer Verbindung der Formel III

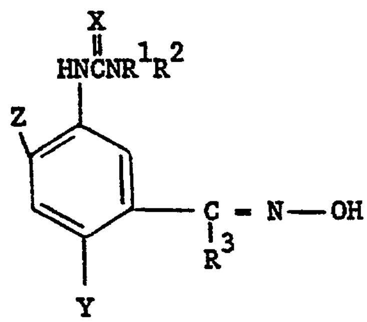

worin $R^1$, $R^2$, $R^3$, X, Y und Z wie oben definiert sind, mit Dihydropyran oder Dihydrofuran umfaßt.

2. Verfahren nach Anspruch 1, worin die Umsetzung bei Umgebungstemperatur in einem inerten Lösungsmittel in der Gegenwart von Säure ausgeführt wird.

3. Verfahren nach Anspruch 1 oder 2, worin in den Formeln I und III X für O steht.

4. Verfahren nach einem der Ansprüche 1 bis 3, worin in den Formeln I und III $R^1$ für Methyl oder Methoxy steht und $R^2$ Methyl ist.

5. Verbindung nach einem der Ansprüche 1 bis 4, worin in den Formeln I und III Y für Wasserstoff oder Brom steht und Z Wasserstoff ist.

6. Verbindung nach einem der Ansprüche 1 bis 5, worin in den Formeln I und III $R^3$ für Methyl steht.

7. Verbindung nach einem der Ansprüche 1 bis 6, worin in der Formel I n den Wert 2 besitzt.

8. Verfahren zur Bekämpfung von unerwünschtem Pflanzenwuchs an einem Ort, welches das Aufbringen einer herbizid wirksamen Menge einer Verbindung der Formel I, wie in einem der Ansprüche 1 und 3 bis 6 definiert, oder einer herbiziden Zusammensetzung, welche eine herbizid wirksame Menge einer Verbindung der Formel I und mindestens ein oberflächenaktives Mittel und/oder einen Träger beinhaltet, auf den Ort umfaßt.

9. Verfahren nach Anspruch 8, worin das Bekämpfen von unerwünschtem Pflanzenwuchs die selektive Bekämpfung von Unkräutern in Baumwolle ist.